# FASCICULE DE BREVET EUROPEEN

(11) **EP 1 786 497 B1**
(45) Date de publication et mention de la délivrance du brevet: **27.03.2013**
(21) Numéro de dépôt: 05792448.2
(22) Date de dépôt: 25.07.2005
(51) Int. Cl.: A61M 5/50

(54) **DISPOSITIF D'INJECTION COMPRENANT UNE SERINGUE**
INJEKTIONSVORRICHTUNG MIT SPRITZE
INJECTION DEVICE COMPRISING A SYRINGE

(30) Priorité: 27.08.2004 FR 0409162
(43) Date de publication de la demande: 23.05.2007
(73) Titulaire: Tech Group Europe Limited, Dublin 2 (IE)
(72) Inventeur: PESSIN, Olivier, F-69290 Grezieu la Varenne (FR)
(74) Mandataire: Blot, Philippe Robert Emile
(86) Numéro de dépôt international: PCT/FR2005/001926
(87) Numéro de publication internationale: WO 2006/027445

(56) Documents cités:
- EP-A- 0 904 792
- FR-A- 2 837 107
- US-A- 5 803 918
- US-A1- 2002 045 864
- US-A1- 2004 144 668
- US-B1- 6 296 625
- US-B1- 6 416 323

## Description

La présente invention concerne un dispositif de protection d'aiguille comportant :
- une seringue d'injection comprenant :
   - un corps de seringue tubulaire équipé à une extrémité proximale d'un col de retenue faisant saillie radialement vers l'extérieur ; et
   - un piston d'injection monté à coulissement dans le corps en étant introduit dans le corps depuis l'extrémité proximale du corps,
- un dispositif de protection d'aiguille comportant :
   - un support de protecteur délimitant un conduit de réception du corps de seringue, lequel support de protecteur comporte des moyens de retenue axiale du corps par rapport au support de protecteur,
   - un protecteur d'aiguille monté déplaçable par rapport au support de protecteur entre une position rétractée et une position déployée.

L'invention s'applique notamment au domaine des seringues d'injection préremplies à usage unique, destinées en particulier aux injections intramusculaires ou souscutanées.

Ces dispositifs sont destinés à limiter au maximum pour l'utilisateur le risque de piqûre accidentelle post-injection puisqu'une fois l'injection du contenu de la seringue réalisée, l'aiguille de la seringue est retirée du patient et le protecteur est automatiquement amené en position déployée au-delà de l'extrémité piquante de l'aiguille.

Un tel dispositif de protection est décrit notamment dans le document FR 2 837 107.

Dans ce dispositif, le dispositif de protection d'aiguille est rapporté sur le corps de seringue. A cet effet, le dispositif de protection comporte un support de protecteur délimitant un passage cylindrique dans lequel est engagé le corps de seringue. A son extrémité proximale, le support de protecteur comporte des moyens d'enclenchement élastiques pour la retenue du corps de seringue depuis une collerette périphérique disposée à son extrémité distale.

Il est fréquent que pour procéder à une injection intramusculaire, le praticien, après avoir piqué l'aiguille, pratique un « test veine » afin de vérifier qu'il n'a pas piqué dans une veine puisqu'il doit injecter dans un muscle. Pour procéder à ce test, le praticien tire le piston de la seringue vers l'arrière. Si cette traction est trop importante, le piston peut être totalement dégagé du corps de seringue, ce qui rend le dispositif d'injection totalement inutilisable, puisque le liquide contenu dans la seringue est alors en contact avec l'air.

Le dispositif décrit dans le document FR 2 837 107 ne résout pas ce problème de déboîtement du piston lors du corps de seringue en cas de traction excessive sur le piston.

Un tel risque existe également lors d'une reprise de lyophilisat, c'est-à-dire lors de l'aspiration dans le corps de seringue depuis l'aiguille d'un mélange exlemporané préparé immédiatement avant injection.

L'invention a pour but de proposer un dispositif d'injection équipé d'un dispositif de protection d'aiguille rapporté, qui réduit les risques d'extraction du piston hors du corps de seringue par traction sur le piston.

A cet effet, l'invention a pour objet un dispositif d'injection selon la revendication 1.

Suivant d'autres caractéristiques de ce dispositif, prises isolément ou selon toutes les combinaisons techniquement possibles :
- les moyens d'enclenchement élastique comportent des saillies d'encliquetage portées par le support de protecteur, la distance entre lesdites saillies d'encliquetage étant supérieure à la section du corps de seringue pour permettre un engagement libre du corps de seringue dans le support de protecteur ;
- des lumières sont ménagées le long des saillies d'encliquetage pour leur conférer leur élasticité. ;
- le support de protecteur comporte une butée d'appui axial du corps de seringue vers l'extrémité proximale, et l'épingle est liée axialement au support de protecteur vers l'extrémité proximale et forme une butée d'arrêt du col vers l'extrémité proximale, de sorte que le col est confiné axialement entre la butée d'appui et l'épingle ;
- l'épingle présente un contour extérieur circulaire.

L'invention sera mieux comprise à la lecture de la description qui va suivre, donnée uniquement à titre d'exemple et faite en se référant aux dessins sur lesquels :
- la figure 1 est une vue éclatée en perspective d'un dispositif d'injection selon l'invention avant assemblage ;
- la figure 2 est une vue en élévation du dispositif de protection appartenant au dispositif d'injection de la figure 1, en position rétractée ;
- la figure 3 est une vue en coupe selon le plan III-III indiqué sur la figure 2 ;
- la figure 4 est une vue de dessus selon la flèche IV indiquée sur la figure 2 ;
- la figure 5 est une vue en coupe selon le plan indiqué V-V sur la figure 4;
- la figure 6 est une vue suivant le même plan de coupe que celui de la figure 5, du dispositif d'injection selon l'invention avant utilisation ;
- la figure 7 est une vue de dessus selon la flèche VII indiquée sur la figure 6;
- la figure 8 est une vue analogue à celle de la figure 6, le dispositif d'injection étant prêt pour l'utilisation ;
- la figure 9 est une vue identique à celle de la figure 8, le dispositif d'injection ayant son piston tiré en position extrême ;
- la figure 10 est une vue suivant le même plan de coupe que celui de la figure 3, du dispositif d'injection en fin de d'utilisation ;
- la figure 11 est une vue identique à celle de la figure 10, la seringue du dispositif d'injection n'étant pas représentée ;
- la figure 12 est une vue en coupe selon le plan XII-XII indiqué sur la figure 10 ; et
- les figures 13 à 15 illustrent le dispositif d'injection en position déployée, la figure 13 étant une vue analogue à celle de la figure 10, la figure 14 étant une vue en coupe selon le plan XIV-XIV indiqué sur la figure 13 et la figure 15 est une vue identique à celle de la figure 13, la seringue n'étant pas représentée.

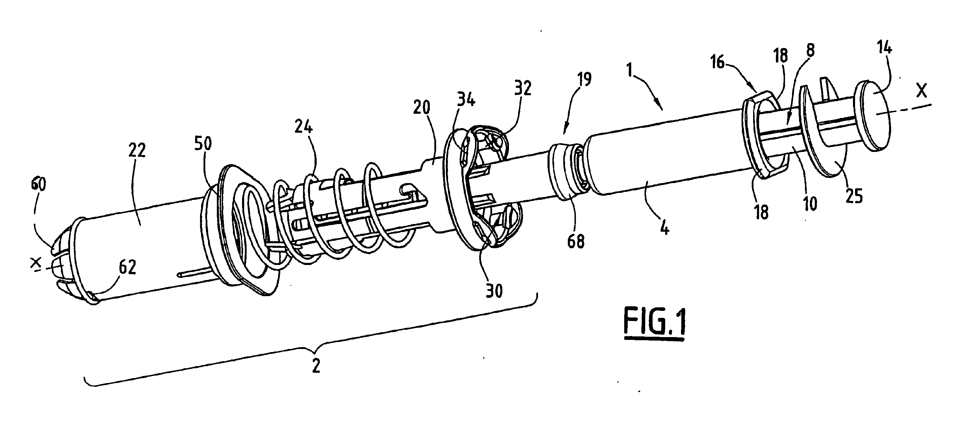

Sur la figure 1 est représenté en perspective un dispositif d'injection formé d'une seringue 1 et d'un ensemble de protection 2. Dans toute la suite, les termes « proximal » et « arrière » sont synonymes, tout comme les termes « distal » et « avant ».

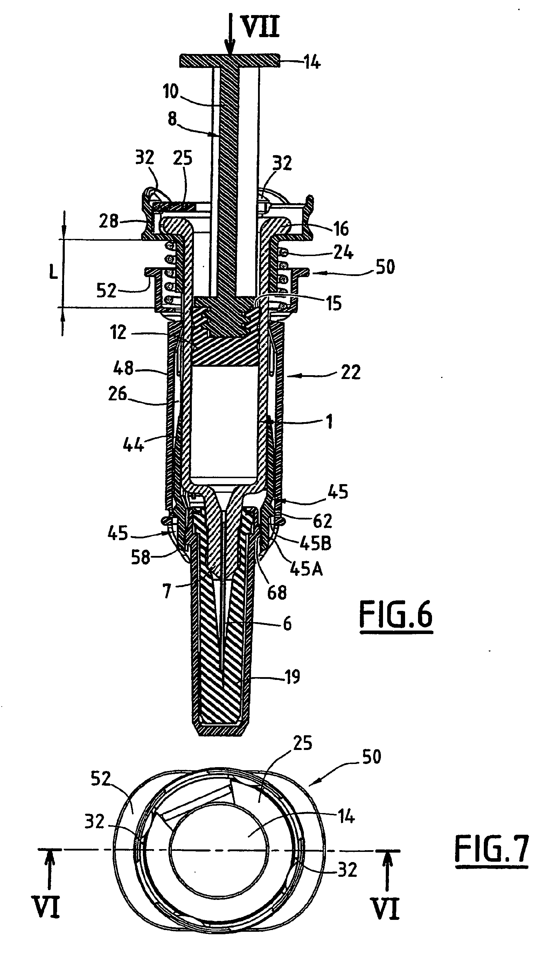

La seringue 1 est une seringue en verre de forme standard, destinée à un usage unique. Elle contient un liquide à injecter, de façon intramusculaire ou sous-cutanée, à un patient. Elle comporte à cet effet un corps 4 et une aiguille 6 visible notamment sur la Figure 6. Cette aiguille est solidaire de l'extrémité distale du corps 4 par un collet 7.

La seringue comporte en outre un piston 8 engagé dans le corps 4. Ce piston comporte classiquement une tige 10 pourvue à son extrémité distale d'un tampon 12 visible sur la figure 6 et d'une tête d'appui 14 sur laquelle est destiné à s'appuyer le pouce de la main du praticien.

La tige 10 a dans sa partie courante une section inférieure à celle du tampon 12 de sorte qu'un épaulement périphérique 15 visible sur la figure 6 est formé sur le tampon 12 autour de la tige 10.

Le corps 4 de la seringue comporte, dans sa partie proximale, un col 16 délimitant circonférentiellement deux oreilles 18 diamétralement opposées, destinées normalement, notamment en l'absence de l'ensemble 2, à former des surfaces d'appui pour l'index et le majeur du praticien lors de la manipulation de la seringue et de l'injection du liquide s'y trouvant.

Initialement, l'aiguille 6 est protégée par un capuchon amovible 19 retenu sur le corps de la seringue en étant engagé sur le collet 7.

L'ensemble de protection 2, d'axe général X-X, comporte essentiellement, comme représenté sur la figure 1 :
- un support 20 de forme générale tubulaire ;
- un manchon protecteur 22 disposé coaxialement au support 20 et de diamètre supérieur à celui du support,
- un ressort 24 ; et
- une épingle 25 de butée du piston et d'accrochage de la seringue dans l'ensemble de protection.

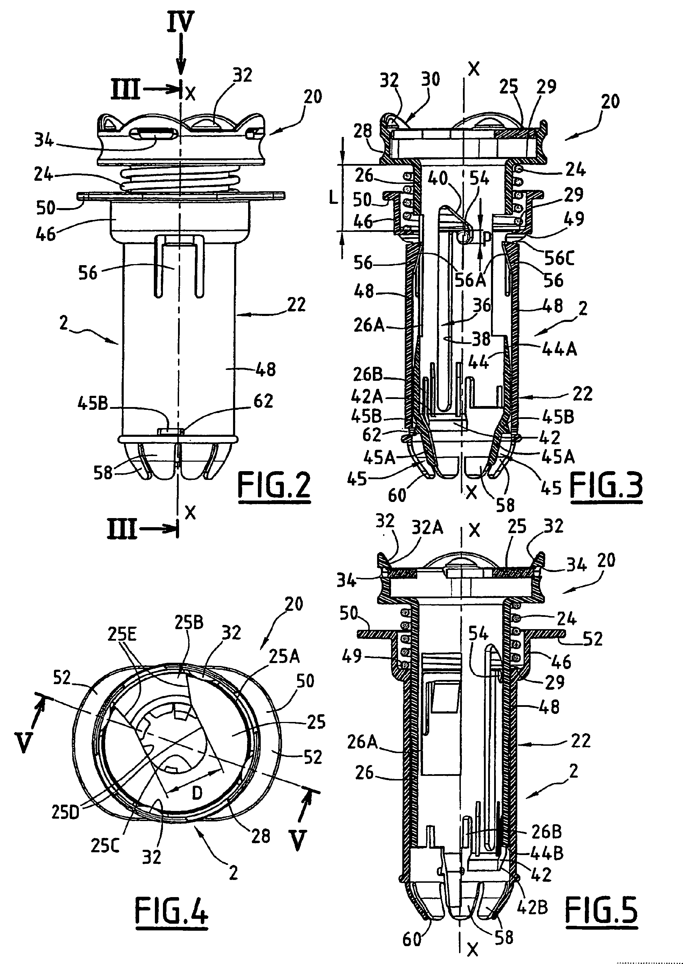

Ces éléments vont successivement être détaillés ci-dessous, en regard des figures 2 à 4.

Le support 20 comporte un tronçon principal 26, sensiblement cylindrique et de diamètre interne sensiblement égal au diamètre externe du corps 4 de la seringue. Ce tronçon 26 présente deux évidements longitudinaux 26A qui sont diamétralement opposés. Le tronçon 26 se prolonge à son extrémité proximale par un tronçon secondaire 28 de plus grands diamètres intérieur et extérieur que ceux du tronçon principal 26, en formant un épaulement radial 29. Le tronçon 28 présente un diamètre intérieur supérieur au diamètre maximal du col 16 de la seringue.

Le tronçon proximal 28 est pourvu de moyens 30 de retenue de l'épingle 25 pour que celle-ci enserre le col de seringue 16 entre l'épaulement 29 et l'épingle 25 comme illustré sur la figure 6.

L'épingle 25 visible sur la Figure 4 est plane et présente une forme de disque évidé pour créer pour former un U. Elle est délimitée à sa périphérie par un contour circulaire 25A de diamètre très légèrement inférieur au diamètre intérieur du tronçon secondaire 28. Elle présente une encoche 25B s'engageant dans le disque formant l'épingle au-delà du centre de celui-ci. Cette encoche 25B débouche radialement à la périphérie du disque formant l'épingle. Elle présente un fond semi-circulaire 25C et deux rives parallèles 25D prolongeant le fond 25C. Ces rives 25D sont prolongées jusqu'à la périphérie 25A du disque par des pans divergeant 25E de sorte que l'encoche 25B s'évase au voisinage de son extrémité débouchante.

Le diamètre du fond 25C et la distance séparant les rives 25D, tous deux désignés par D, sont choisis supérieurs au diamètre de la partie courante de la tige de piston 10, et inférieurs au diamètre du tampon 12 prolongeant la tige 10. Ainsi, ce diamètre D est inférieur à la section intérieure du corps de seringue 4.

Les moyens de retenue 30 comportent des crochets déformables 32 diamétralement opposés qui sont portés par le tronçon 28 du support de protecteur. Ces crochets sont visibles notamment sur les figures 1 et 5. La distance séparant les crochets diamétralement opposés est inférieure au diamètre extérieur de l'épingle 25. Des lumières 34 sont ménagées dans le tronçon 28 le long des crochets 32 pour conférer à ceux-ci une élasticité.

Chacun de ces crochets ménage une rampe 32A sensiblement tronconique s'évasant vers l'extrémité libre du tronçon 28. Ces rampes 32A sont destinées à permettre de repousser élastiquement les crochets vers l'extérieur sous l'action de l'épingle 25 lors de la fixation du support 20 sur la seringue 1. Les crochets 32 sont distants de l'épaulement 29 d'une distance sensiblement égale aux épaisseurs cumulées des oreilles 18 et de l'épingle 25. Les crochets 32 forment ainsi un moyen de clipsage de l'épingle 25 pour la retenue du col de seringue 16.

Deux rainures traversantes 36 en forme de crosse sont ménagées l'une en face de l'autre dans le tronçon principal 26. Chaque rainure est constituée d'une première partie rectiligne 38 qui s'étend sensiblement suivant l'axe X-X du support 20 sur une longueur supérieure à celle de l'aiguille 6, et d'une seconde partie rectiligne 40 qui s'étend de manière inclinée par rapport au même axe X-X. La partie inclinée 40 débouche à l'extrémité proximale de la première partie rectiligne 38, en formant un V dont la pointe est dirigée du côté proximal de l'ensemble 2.

Le tronçon principal 26 comporte à son extrémité distale une première paire de languettes élastiques 42 diamétralement opposées, situées chacune dans le prolongement des rainures 36 (figure 5). Ces languettes 42 présentent une face interne 42A de forme sensiblement cylindrique et une face externe 42B sensiblement tronconique, divergente vers l'arrière.

Le tronçon principal 26 comporte une paire de rampes externes 44 diamétralement opposées, situées entre les languettes élastiques 42 en suivant la circonférence de l'extrémité distale de ce tronçon. Elles présentent une surface externe inclinée 44A sensiblement tronconique, divergente vers l'avant, et une surface distale 44B sensiblement plane. Les surfaces externes 44A sont ainsi tournées vers les évidements 26A.

Les évidements longitudinaux 26A sont ménagés dans le tronçon principal 26 (figure 3) à l'extrémité proximale de ces rampes 44.

Par ailleurs, de part et d'autre des languettes 42, des fentes axiales 26B sont prévues à partir de l'extrémité distale du support, de sorte que, avant insertion du corps 4 de la seringue 1 à l'intérieur du support 20, ces languettes 42 sont radialement déformables, notamment vers l'intérieur.

A son extrémité distale ou avant, le tronçon principal 26 présente, entre les languettes élastiques 42, deux autres languettes 45 de solidarisation axiale du protecteur 22 et du support de protecteur 20 en présence du capuchon de protection 19 placé sur le corps de seringue.

Plus précisément et comme illustré sur la Figure 3, ces languettes comportent chacune une jambe 45A prolongeant le tronçon principal 26 et venue de matière avec celui-ci. Chaque jambe 45A porte extérieurement une saillie 45B propre à s'engager dans une lumière complémentaire ménagée dans le protecteur.

Les saillies 45B sont prévues dans la moitié de la longueur des jambes 45A s'étendant depuis la région de liaison des jambes au tronçon principal 26. Ainsi, les jambes 45A se prolongent au-delà des saillies 45B.

Comme illustré sur la Figure 3, les languettes sont, au repos, inclinées vers l'axe X-X du support, c'est-à-dire qu'elles convergent l'une vers l'autre en direction de leur extrémité libre et qu'elles s'étendent dans le prolongement du passage intérieur délimité par le support de protecteur.

Ces languettes 45 sont déformables élastiquement vers l'extérieur de sorte que, en position de repos, les saillies sont complétement contenues dans le prolongement de l'encombrement général du tronçon principal 26 alors que, en position déformée, les saillies 45B s'étendent radialement vers l'extérieur au-delà du prolongement de l'encombrement général du tronçon principal 26.

La distance séparant les extrémités libres des deux languettes 45, lorsqu'elles sont dans leur position de repos est inférieure au diamètre extérieure minimal de la bague de retenue du capuchon protecteur 19 de seringue.

Le manchon protecteur 22 est de longueur sensiblement égale à celle du corps 4 de la seringue. Il est constitué de deux tronçons cylindriques 46 et 48, le tronçon proximal 46 étant de diamètre légèrement supérieur à celui du tronçon principal 48. Ces deux tronçons se raccordent en formant un épaulement radial 49.

Le manchon 22 comporte solidairement, dans sa partie proximale, une collerette extérieure 50 sous forme de deux oreilles 52 diamétralement opposées (figures 4 et 5).

Egalement dans sa partie proximale, mais à l'intérieur du manchon protecteur 22, deux tétons 54 diamétralement opposés sont solidaires du manchon (figure 5). Ces deux tétons sont reçus et guidés dans respectivement les deux rainures 36 du support. Le support et le manchon sont ainsi mobiles l'un par rapport à l'autre en translation suivant leur axe commun et en rotation limitée autour du même axe lorsque les tétons se trouvent dans les parties inclinées 40. Les parties inclinées 40 forment alors des poches de rétention des tétons 54, ces poches ayant une profondeur de rétention notée p sur la Figure 3. Cette profondeur est mesurée suivant l'axe du protecteur.

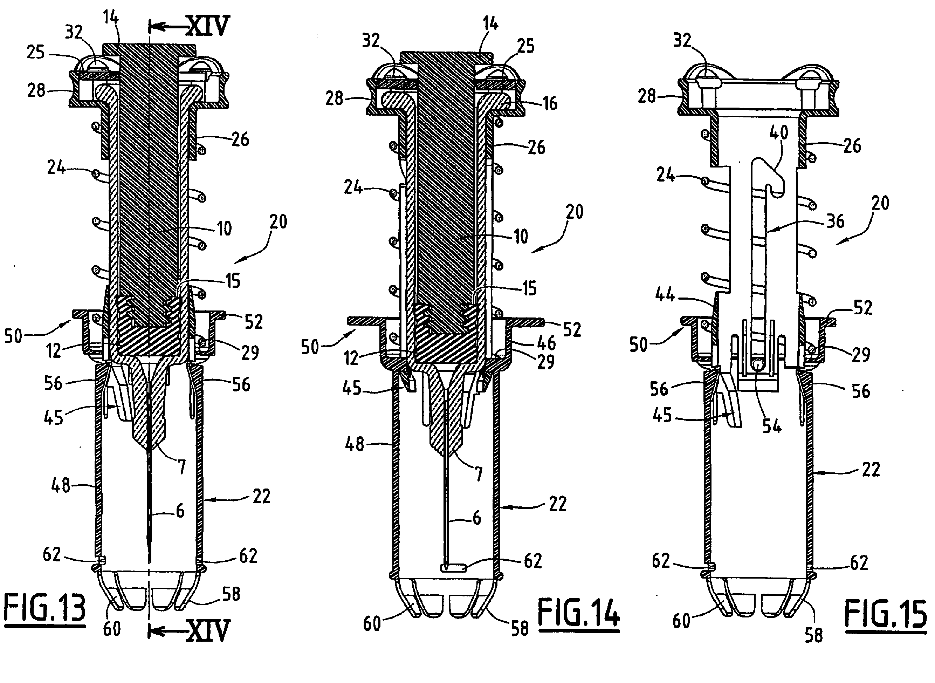

Le support 20 et le manchon 22 sont mobiles entre une position rétractée du manchon, dans laquelle l'essentiel du manchon recouvre l'essentiel du support et les tétons 54 sont situés à l'extrémité distale de chacune des parties de rainures inclinées 40, comme représenté sur les figures 2 à 8, et une position déployée du manchon dans laquelle celui-ci s'étend axialement en saillie du support et les tétons sont situés à l'extrémité distale de la partie de rainure rectiligne 38, comme représenté sur les figures 13 à 15.

Lorsque la seringue 1 est fixée sur l'ensemble 2, ces positions extrêmes correspondent respectivement à une configuration d'injection dans laquelle l'aiguille 6 de la seringue 1 est dégagée et destinée à être insérée dans un patient, et à une configuration de protection dans laquelle cette aiguille est entourée du manchon protecteur 22.

La partie proximale du manchon 22 comporte en outre intérieurement une paire de crochets longitudinaux déformables 56 diamétralement opposés. Ces crochets sont délimités dans le manchon 22 par des fentes latérales. Ils sont liés au manchon à leur extrémité distale. Chaque crochet présente à son extrémité libre proximale une protubérance intérieure.

En l'absence de seringue, et comme illustré sur la Figure 3, les surfaces externes des crochets 56 s'étendent dans le prolongement du manchon. Au contraire, les protubérances internes de ces crochets font saillie à l'intérieur du passage cylindrique délimité par le manchon 22. Chaque protubérance présente une face avant 56A de forme sensiblement tronconique s'écartant vers l'avant de l'axe du manchon 22. Ces faces avant 56A sont ainsi tournées vers l'extrémité avant du protecteur.

Chaque face avant 56A est adaptée pour coopérer avec une surface inclinée 44A formée par les rampes 44 du support.

A son extrémité libre, chaque crochet 56 présente un front transversal incliné 56C formant butée.

Dans la position rétractée du manchon, les crochets 56 s'étendent à l'intérieur des évidements 26A ménagés dans le support 20. Dans la position déployée du manchon, comme représenté sur la figure 13, les faces d'extrémité 56C des crochets 56 sont en butée axiale contre les languettes 42, les crochets et languettes formant de la sorte un ensemble de blocage rigide en position déployée.

Le manchon 22 est par ailleurs pourvu à son extrémité distale d'une couronne de languettes déformables 58, dont les bords distaux forment une ouverture 60 sensiblement circulaire, de diamètre inférieur au diamètre intérieur du tronçon principal 26 du support 20.

Enfin, à son extrémité distale, le tronçon principal 48 du manchon protecteur comporte deux lumières oblongues traversantes 62 propres à recevoir les saillies 45B portées par la seconde paire de languettes 45 lorsque celles-ci sont déformées sous l'action de la bague du capuchon 19 de protection d'aiguille engagé sur une seringue contenue dans le dispositif.

En l'absence de capuchon, alors que les languettes 45 sont dans leur position de repos, les saillies 45B sont totalement hors des lumières 62 permettant un déplacement libre du protecteur par rapport au support.

Le ressort 24 est un ressort spiralé, disposé entre le manchon protecteur 22 et le support de protecteur 20. Plus précisément, le ressort est logé entre l'épaulement 29 du support 20 et l'épaulement 49 du manchon 22.

Dans la position rétractée du manchon, le ressort 24 est dans un état comprimé, possédant ainsi une énergie de décompression liée à la raideur du ressort et à la différence entre la longueur du ressort à l'état au repos et sa longueur, notée L sur les figures 2 à 8, à l'état comprimé. Cela revient à dire que le ressort 24 présente un seuil de force de compression supplémentaire qui correspond à la force minimale nécessaire pour comprimer davantage le ressort à partir de son état comprimé initial des figures 2 à 8. La raideur du ressort et/ou la longueur de compression initiale L sont choisies de telle sorte que ce seuil de force soit supérieur à l'effort de poussée nécessaire pour déplacer le piston 8 de la seringue 1 sur toute sa course d'injection. Plus précisément, la force du ressort à l'état verrouillé est supérieure à la somme de l'effort d'injection, c'est-à-dire d'évacuation du liquide à l'extérieur de l'aiguille 6 de la seringue 1, et des efforts de décollement et de glissement du poussoir 12 à l'intérieur du corps 4 de la seringue.

Le capuchon 19 est de forme générale tubulaire, et est représenté sur les figures 1 et 6.

Il est adapté pour entourer l'aiguille 6 avant utilisation de la seringue 1. Ce capuchon est fermé à une de ses extrémités et son extrémité opposée est formée par une bague annulaire 68 de diamètre extérieur adapté pour à la fois être conjugué de la surface 42A des languettes 42 et être supérieur au diamètre de l'ouverture 60 formée par les languettes 58 du manchon protecteur 22. La face intérieure de cette bague 68 est destinée à adhérer sur le collet en verre 7 du corps de seringue où est fixée l'aiguille 6, notamment pour garantir une certaine étanchéité aux bactéries.

En outre, le diamètre extérieur de la bague 68 du capuchon est supérieur à la distance séparant au repos les extrémités libres des languettes 45.

Le fonctionnement du dispositif d'injection selon l'invention est le suivant.

Le dispositif de protection 2 est assemblé dans sa configuration rétractée, c'est-à-dire celle des figures 2 à 8. Le manchon protecteur 22 est pour cela enfilé autour du support 20 à partir de l'extrémité distale du support, en disposant entre eux le ressort 24. Plus précisément, on déplace axialement vers l'arrière le manchon 22 par rapport au support, tout en déformant radialement vers l'extérieur les crochets 56 au moyen d'un outil adapté, et ce au moins jusqu'à ce qu'ils atteignent axialement la partie avant des évidements longitudinaux 26A.

Puis toujours en déplaçant le manchon vers l'arrière, les tétons 54 sont appliqués contre les surfaces externes 42B des languettes 42, en déformant ces dernières vers l'intérieur, jusqu'à ce que les pions soient reçus dans les parties rectilignes 38. Le déplacement du protecteur 22 vers l'arrière se poursuit ensuite jusqu'à ce que les tétons 54 soient reçus dans les parties de rainures inclinées 40, en faisant pivoter l'un par rapport à l'autre le support et le protecteur. Le protecteur se retrouve ainsi en position rétractée.

La seringue en verre 1 est préremplie d'un liquide à injecter dans un patient. Cette seringue est équipée du capuchon 19 qui enserre le collet 9 du corps de seringue 4.

La seringue équipée du capuchon 19 est insérée à l'intérieur de l'ensemble 2 pour former le dispositif d'injection, tel que représenté sur les figures 6 et 7. Plus précisément, le corps 4 de la seringue est déplacé sensiblement axialement à l'intérieur du support 20.

Le déplacement du corps de seringue dans le protecteur est effectué jusqu'à ce que le col de seringue 16 s'appuie sur l'épaulement 29.

Dans cette position, l'épingle 25 est alors rapportée sur le col 16. A cet effet, si la tige de piston 10 est déjà montée sur le tampon 12, l'épingle 25 est engagée autour de la tige de piston, celle-ci se trouvant dans l'encoche. L'épingle 25 est déplacée suivant la longueur de la tige 10 puis est engagée dans le tronçon principal 28 en déformant radialement vers l'extérieur les crochets 32 de manière à assurer un enclenchement élastique de l'épingle.

Les oreilles 18 du col de seringue 16 sont alors retenues axialement par l'épingle 25 elle-même retenue par les crochets 32.

Dans la mesure où le col de seringue 16 est totalement enclipsé à l'intérieur du tronçon 28, il ne peut plus remplir son rôle habituel de formation de surface d'appui pour l'index et le majeur du praticien. Cette fonction d'appui est réalisée par la collerette 50 solidaire du manchon 22. En effet, dans la mesure où la longueur du manchon protecteur 22 est sensiblement égale à celle du corps de seringue 4 et/ou où la collerette 50 est ménagée au niveau de l'extrémité proximale de ce manchon, le praticien peut alors manipuler la seringue en faisant reposer son pouce sur la tête d'appui 14 du piston 8 comme à l'accoutumée, et en faisant reposer son index et son majeur sur les faces des oreilles 52 dirigées vers l'aiguille 6.

Par ailleurs, lorsque la seringue 1 est fixée sur le support de protecteur 20 comme représenté sur les figures 6 et 7, le capuchon 19 s'étend en saillie à l'extérieur du manchon protecteur 22.

Qui plus est, le diamètre extérieur élargi de la bague 68 du capuchon se trouve engagé entre les extrémités libres des languettes 45, ce qui assure ainsi leur déformation radiale vers l'extérieur. Sous l'action de cette déformation, les saillies 45B sont reçues dans les lumières 62. Dans cette position, la coopération des saillies 45B portées par le support de protecteur et des lumières 62 ménagées dans le protecteur assure une solidarisation axiale du protecteur et du support, interdisant ainsi une libération du ressort, le téton 54 ne pouvant quitter l'extrémité de la crosse dans laquelle il est engagé.

Lorsque le praticien est prêt à réaliser l'injection du liquide contenu dans la seringue, il retire le capuchon 19 en le tirant axialement vers l'avant.

Pour retirer le capuchon, le praticien peut maintenir la seringue en retenant le protecteur d'une main depuis les surfaces d'appui 50 et tirer sur le capuchon 19 avec l'autre main. Même si la résistance au retrait du capuchon est très importante, la liaison axiale positive assurée par les saillies 45B engagées dans les lumières 62 évite que le ressort 24 ne puisse être comprimé et que les tétons 54 puissent être dégagés hors de la crosse de retenue.

Ainsi, aucun déclenchement accidentel du protecteur d'aiguille ne peut avoir lieu lors du retrait du capuchon.

En revanche, après retrait du capuchon comme illustré sur la figure 7, le protecteur 22 peut être déplacé par rapport au support 20 puisque les languettes 45 reprennent leur position de repos sous l'action de leur élasticité et les saillies 45A sont alors dégagées des lumières 62.

Après le décollement de la bague 68 du collet 9, la bague 68 franchit l'ouverture 60 en déformant les languettes 58. Une fois le capuchon 19 retiré, les languettes 58 reprennent leur position initiale.

Le praticien peut alors être amené à pratiquer une opération connue sous l'expression « test veine ». Celle-ci consiste à vérifier que la seringue a été correctement piquée dans un muscle du patient et non dans une artère ou une veine. A cet effet, le piston 8 est tiré vers l'extrémité proximale. Si du sang est aspiré dans le corps de seringue, le praticien déduit que l'aiguille est piquée dans une veine ou une artère et il recommence alors la mise en place de l'aiguille.

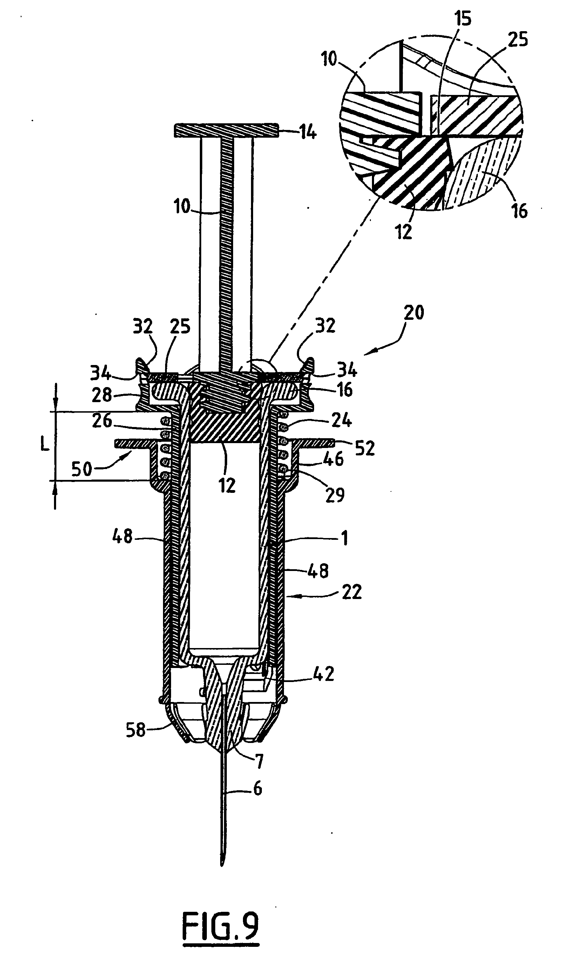

En cas de traction excessive exercée par le praticien sur la tige de piston, le tampon 12 est arrêté dans son déplacement en butant par l'intermédiaire de l'épaulement 15 contre l'épingle 25 comme illustré sur la figure 9. En effet, l'encoche 25B définit un passage de section inférieure à la section du tampon 12 de sorte que celui-ci ne peut passer au-delà de l'épingle. En revanche, la tige de piston 10 ayant un diamètre inférieur, celui-ci peut coulisser librement au travers de l'encoche 25B.

Ainsi, on conçoit que l'épingle 25 assure à la fois la retenue du corps de seringue 4 dans le protecteur et d'autre part constitue une butée d'arrêt du tampon 12 en cas de traction excessive sur celui-ci. En l'absence de l'épingle, le tampon pourrait être extrait du corps de seringue, rendant ainsi le dispositif d'injection inutilisable.

L'arrêt du piston en cas de traction excessive sur celui-ci est également utile lors du remplissage de la seringue au travers de l'aiguille par exemple lors d'une reprise de lyophilisat.

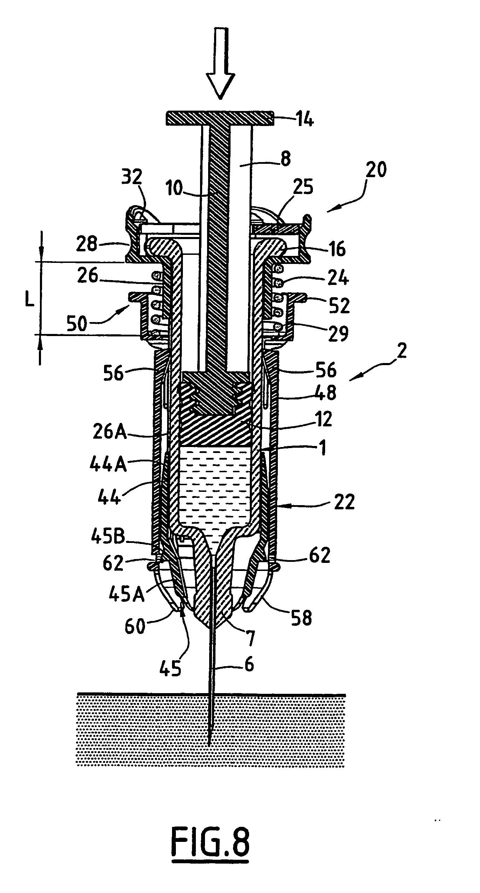

Pour procéder à l'injection proprement dite, le praticien expulse le liquide contenu dans la seringue en exerçant une poussée sur la tête d'appui 14 du piston, son index et son majeur demeurant en contact avec les faces dirigées vers l'aiguille des oreilles 52. Durant l'injection, aucun mouvement ne se produit entre le support de protecteur 20 et le manchon protecteur 22, le ressort 24 demeurant comprimé avec une longueur L, comme représenté sur la figure 8.

L'injection se poursuit jusqu'à ce que le poussoir 12 du piston 8 parvienne en fin de course d'injection.

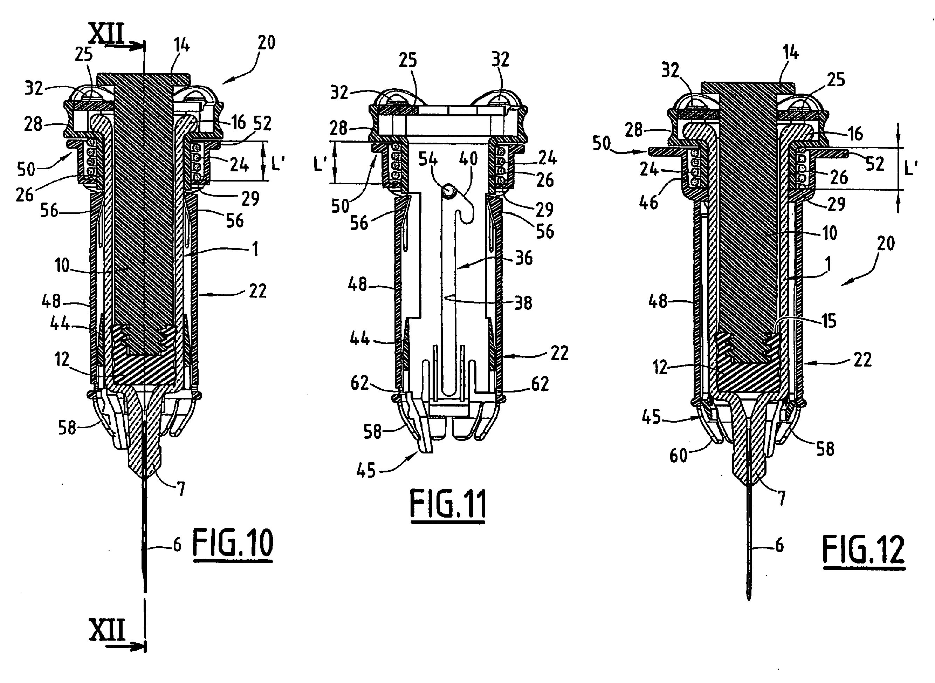

Le praticien retire alors l'aiguille du patient. Pour déclencher l'ensemble de protection 2, le praticien exerce une pression supplémentaire sur la tige de piston 8. Cette pression doit être supérieure à la force prédéterminée produite par le ressort 24 à l'état verrouillé, de sorte que ce ressort est davantage comprimé et passe de sa longueur L à une longueur L' plus petite, comme représenté sur les figures 10 à 12. Pour ce faire, en raisonnant à support de seringue immobile, le manchon protecteur 22 se déplace axialement vers l'extrémité proximale du support 20. Le praticien réalise ce mouvement en exerçant une pression correspondante au moyen de son index et de son majeur sur les oreilles 52 de la collerette 50 du manchon 22. Cette pression entraîne, de façon combinée au mouvement de translation, la rotation du manchon protecteur 22 autour du support de seringue 20, les tétons 54 étant guidés par les parties de rainure inclinées 40. Ce mouvement de rotation se poursuit jusqu'à ce que les tétons atteignent l'extrémité proximale de cette partie de rainure 40, c'est-à-dire l'extrémité proximale de la partie de rainure longitudinale 38, comme visible sur la figure 11. Le dispositif 2 est alors en position de déverrouillage du ressort 24.

Le praticien relâche ensuite la pression qu'il exerçait jusqu'alors sur la collerette 50, permettant au ressort 24 de se détendre jusqu'à un état de repos. Les tétons 54 se déplacent en translation à l'intérieur de la partie de rainure longitudinale 38, jusqu'à l'extrémité distale de celle-ci comme représenté sur les figures 13 à 15. Le mouvement de translation du manchon protecteur 22 par rapport au support 20 est contrôlable par le praticien, si ce dernier relâche progressivement la retenue digitale qu'il exerce la collerette 50. Lorsque les tétons 54 sont arrivés à l'extrémité distale de la rainure 36 (figure 15), le protecteur est dans sa position déployée.

Par ailleurs, lorsque le manchon protecteur 22 est en mouvement de translation par rapport au support 20, les crochets 56 empruntent les évidements longitudinaux 26A du support, jusqu'à ce qu'ils glissent le long des rampes distales 44 du support, par coopération de leurs surfaces complémentaires 56A et 44A.

En position déployée du protecteur, les crochets 56 sont maintenus par coopération des surfaces 56C et 44B, de sorte que le manchon protecteur 22 ne peut pas être ramené dans sa position initiale. De même, le manchon 22 ne peut pas être facilement arraché du support 20 puisque les tétons 54 sont en butée contre le fond distal de la partie de rainure longitudinale 38 (figure 15), les languettes 42 formant ce fond étant radialement maintenues entre le corps de la seringue 1 et le manchon protecteur 22.

Le dispositif d'injection selon l'invention est ainsi simple d'utilisation, tout en permettant au praticien de contrôler le mouvement de recouvrement de l'aiguille par le manchon protecteur. Le nombre de pièces dont est constitué l'ensemble de protection 2 représenté est réduit à trois.

Le dispositif selon l'invention est adaptable à différents types de seringues, tant au niveau des formes qu'à celui des volumes. Ce dispositif présente donc l'avantage de ne pas remettre en cause la forme générale des seringues utilisées et par conséquent n'entraîne aucune modification des procédés industriels de remplissage de ces seringues.

Diverses variantes du dispositif selon l'invention sont envisageables :
- à la différence de l'exemple de réalisation décrit ci-dessus, les tétons 54 et/ou la collerette 50 du manchon protecteur 22 peuvent être rapportés sur le manchon 22 et non réalisés d'un seul tenant avec celui-ci;
- à l'inverse du dispositif décrit, les tétons 54 peuvent être réalisés sur la surface externe du support de protecteur 20 et la rainure de guidage 36 ménagée dans le manchon protecteur 22 ; et/ou
- le support 20 peut être réalisé d'une seule pièce avec le corps de seringue 4.

## Revendications

1. Dispositif d'injection comportant :
- une seringue d'injection (1) comprenant :
• un corps de seringue tubulaire (4) équipé à une extrémité proximale d'un col (16) de retenue faisant saillie radialement vers l'extérieur ; et
• un piston d'injection (8) monté à coulissement dans le corps (4) en étant introduit dans le corps depuis l'extrémité proximale du corps (4),
- un dispositif (2) de protection d'aiguille comportant :
• un support de protecteur (20) délimitant un conduit de réception du corps de seringue (4), lequel support de protecteur (20) comporte des moyens (30) de retenue axiale du corps (4) par rapport au support de protecteur (20),
• un protecteur d'aiguille (22) monté déplaçable par rapport au support de protecteur (20) entre une position rétractée et une position déployée,
**caractérisé en ce que** :
- le piston (8) comporte une extrémité distale (14) engagée dans le corps de seringue (4) et prolongée vers l'extrémité proximale par une partie courante (10) du piston, un épaulement (15) tourné vers l'extrémité proximale étant défini entre l'extrémité distale (14) et la partie courante (10) du piston, et
- le dispositif d'injection comporte une épingle (25) de butée pour l'arrêt du piston (8), laquelle épingle (25) est portée par le support de protecteur (20) et définit, dans le prolongement du corps de seringue (4), un passage (25B) de circulation du piston (8) dont la section minimale est supérieure à la section de la partie courante (10) du piston (8) et est inférieure à la section de l'épaulement (15) du piston,
- l'épingle (25) délimite une encoche (25B) s'ouvrant radialement et dont le fond (25C) délimite ledit passage de circulation du piston (8), et
- le support de protecteur (20) comporte des moyens (32) d'enclenchement élastique de l'épingle (25) pour sa retenue axiale.

2. D spositif d'injection selon la revendication 1, **caractérisé en ce que** la distance séparant les rives (25D) délimitant l'encoche est choisie supérieure au diamètre de la partie courante du piston (10) et inférieure à la section intérieure du cops de seringue (4).

3. Dispositif d'injection suivant la revendication 1 ou 2, **caractérisé en ce que** les moyens d'enclenchement élastique comportent des saillies d'encliquetage (32) portées par le support de protecteur (20), la distance entre lesdites saillies d'encliquetage (32) étant supérieure à la section du corps de seringue pour permettre un engagement libre du corps de seringue (4) dans le support de protecteur (20).

4. Dispositif d'injection selon la revendication 3, **caractérisé en ce que** des lumières (34) sont ménagées le long des saillies d'encliquetage (32) pour leur conférer leur élasticité.

5. Dispositif d'injection suivant l'une quelconque des revendications précédentes, **caractérisé en ce que** le support de protecteur (20) comporte une butée (29) c'appui axial du corps de seringue (4) vers l'extrémité proximale, et **en ce que** l'épingle (25) est liée axialement au support de protecteur (20) vers l'extrémité proximale et forme une butée d'arrêt du col (16) vers l'extrémité proximale, de sorte que le col (16) est confiné axialement entre la butée d'appui (29) et l'épingle (25).

6. Dispositif d'injection suivant l'une quelconque des revendications précédentes, **caractérisé en ce que** l'épingle (25) présente un contour extérieur circulaire.

## Claims

1. Injection device comprising:
- an injection syringe (1) comprising:
• a tubular syringe body (4) equipped at a proximal end with a fixing collar (16) projecting radially outward; and
• an injection plunger (8) mounted to slide within the body (4) by being introduced into the body from the proximal end of the body (4),
- a needle protection device (2) comprising:
• a protector support (20) delimiting a conduit for receiving the syringe body (4), which protector support (20) comprises means (30) for axially fixing the body (4) relative to the protector support (20),
• a needle protector (22) mounted so as to be displaceable relative to the protector support (20) between a retracted position and an extended position,
**characterized in that**:
- the plunger (8) comprises a distal end (14) engaged in the syringe body (4) and extended toward the proximal end by a common portion (10) of the plunger, a shoulder (15) turned toward the proximal end being defined between the distal end (14) and the common portion (10) of the plunger, and
- the injection device comprises a stop clip (25) for stopping the plunger (8), which clip (25) is carried by the protector support (20) and defines, in the extension of the syringe body (4), a travel passage (25B) for the plunger (8), the minimum section of which is greater than the section of the common portion (10) of the plunger (8) and is smaller than the section of the plunger shoulder (15),
- the clip (25) delimits a notch (25B) which opens radially and the base (25C) of which delimits said travel passage for the plunger (8), and
- the protector support (20) comprises means (32) for resiliently interlocking the clip (25) for the axial fixing thereof.

2. Injection device according to claim 1, **characterised in that** the distance separating the lips (25D) delimiting the notch is chosen to be greater than the diameter of the common portion (10) of the plunger and smaller than the internal section of the syringe body (4).

3. Injection device according to claim 1 or 2, **characterised in that** the resilient interlocking means comprise click-lock projections (32) carried by the protector support (20), the distance between said click-lock projections (32) being greater than the section of the syringe body to allow free engagement of the syringe body (4) with the protector support (20).

4. Injection device according to claim 3, **characterised in that** orifices (34) are formed along the click-lock projections (32) to impart resilience thereto.

5. Injection device according to any if the preceding claims, **characterised in that** the protector support (20) comprises a stop (29) for axially supporting the syringe body (4) toward the proximal end and **in that** the clip (25) is axially connected to the protector support (20) toward the proximal end and forms a stop for stopping the collar (16) toward the proximal end so that the collar (16) is axially confined between the support stop (29) and the clip (25).

6. Injection device according to claim 1, **characterised in that** the clip (25) has a circular outer contour.

## Patentansprüche

1. Injektionsvorrichtung, Folgendes umfassend:
- eine Injektionsspritze (1), die aufweist:
• einen rohrförmigen Spritzenkörper (4), der an einem proximalen Ende mit einem Halteansatz (16) ausgestattet ist, der radial nach außen vorsteht; und
• einen Injektionskolben (8), der verschiebbar im Körper (4) angebracht ist, wobei er vom proximalen Ende des Körpers (4) her in den Körper eingeführt ist,
- eine Nadelschutzeinrichtung (2), die Folgendes umfasst:
• einen Schutzträger (20), der einen Aufnahmekanal des Spritzenkörpers (4) abgrenzt, welcher Schutzträger (20) Mittel (30) zum axialen Halten des Körpers (4) in Bezug auf den Schutzträger (20) umfasst,
• eine Nadelschutzvorrichtung (22) die in Bezug auf den Schutzträger (20) zwischen einer eingefahrenen Position und einer ausgefahrenen Position verlagerbar ist,
**dadurch gekennzeichnet, dass**:
- der Kolben (8) ein distales Ende (14) umfasst, das in den Spritzenkörper (4) eingesteckt und zum proximalen Ende hin durch einen gängigen Abschnitt (10) des Kolbens verlängert ist, wobei eine zum proximalen Ende hin gewandte Schulter (15) zwischen dem distalen Ende (14) und dem gängigen Abschnitt (10) des Kolbens definiert ist, und
- die Injektionsvorrichtung einen Anschlagstift (25) zum Stoppen des Kolbens (8) umfasst, welcher Stift (25) vom Schutzträger (20) getragen ist und in der Verlängerung des Spritzenkörpers (4) einen Zirkulationsdurchgang (25B) des Kolbens (8) definiert, dessen Mindestquerschnitt größer als der Querschnitt des gängigen Abschnitts (10) des Kolbens (8) und kleiner als der Querschnitt der Schulter (15) des Kolbens ist,
- der Stift (25) eine Ausklinkung (25B) abgrenzt, die sich radial öffnet, und deren Grund (25C) den Zirkulationsdurchgang des Kolbens (8) abgrenzt, und
- der Schutzträger (20) elastische Einrastmittel (32) des Stifts (25) für dessen axialen Halt umfasst.

2. Injektionsvorrichtung nach Anspruch 1, **dadurch gekennzeichnet, dass** der Abstand der die Ausklinkung abgrenzenden Ränder (25D) größer als der Durchmesser des gängigen Abschnitts des Kolbens (10) und kleiner als der Innenquerschnitt des Spritzenkörpers (4) gewählt ist.

3. Injektionsvorrichtung nach Anspruch 1 oder 2, **dadurch gekennzeichnet, dass** die elastischen Einrastmittel Einrastvorsprünge (32) umfassen, die vom Schutzträger (20) getragen sind, wobei der Abstand zwischen den Einrastvorsprüngen (32) größer ist als der Querschnitt des Spritzenkörpers, um ein freies Einstecken des Spritzenkörpers (4) in den Schutzträger (20) zuzulassen.

4. Injektionsvorrichtung nach Anspruch 3, **dadurch gekennzeichnet, dass** Öffnungen (34) entlang den Einrastvorsprüngen (32) ausgebildet sind, um ihnen Elastizität zu verleihen.

5. Injektionsvorrichtung nach einem der vorhergehenden Ansprüche, **dadurch gekennzeichnet, dass** der Schutzträger (20) einen Anschlag (29) zur axialen Anlage des Spritzenkörpers (4) zum proximalen Ende hin umfasst, und dass der Stift (25) zum proximalen Ende hin axial mit dem Schutzträger (20) verbunden ist und einen Stoppanschlag für den Kragen (16) zum proximalen Ende hin bildet, so dass der Ansatz (16) axial zwischen dem Anlageanschlag (29) und dem Stift (25) eingeschlossen ist.

6. Injektionsvorrichtung nach einem der vorhergehenden Ansprüche, **dadurch gekennzeichnet, dass** der Stift (25) eine kreisförmige Außenkontur aufweist.
